# EUROPEAN PATENT SPECIFICATION

(11) **EP 3 787 551 B1**
(45) Date of publication and mention of the grant of the patent: **25.09.2024**
(21) Application number: 18917199.4
(22) Date of filing: 02.11.2018
(51) Int. Cl.: A61F 2/00, A61F 2/60, A61F 2/66

(54) **PROSTHETIC FOOT WITH SPACED SPRING ELEMENTS**
PROTHESENFUSS MIT BEABSTANDETEN FEDERELEMENTEN
PIED PROTHÉTIQUE À ÉLÉMENTS DE RESSORT ESPACÉS

(30) Priority: 30.04.2018 US 201862664538 P
(43) Date of publication of application: 10.03.2021
(73) Proprietor: Otto Bock HealthCare LP, Minneapolis, MN 55447 (US)
(72) Inventor: DAY, Jesse, Utah 84117 (US); SWEETGALL, Joanna, Salt Lake City, Utah 84102 (US); HALL, Randy, Riverton, Utah 84096 (US); RAY, Darin, Centerville, Utah 84014 (US); MOENICKE, Carsten, 37115 Duderstadt (DE)
(74) Representative: Gramm, Lins & Partner Patent- und Rechtsanwälte PartGmbB
(86) International application number: PCT/US2018/058848
(87) International publication number: WO 2019/212593

(56) References cited:
- WO-A1-00/27317
- US-A1- 2003 191 541
- US-A1- 2012 271 434
- US-A1- 2015 003 745
- US-A1- 2016 081 821
- US-B1- 6 241 776
- US-B2- 8 007 544

## Description

The present disclosure relates generally to prosthetic devices, and more particularly relates to prosthetic feet having improved force absorption.

Prosthetic feet serve as distal termination for a prosthetic device and can be fixed to a below knee tube, which is fastened to a prosthetic knee joint, directly to a prosthetic shank or to the prosthetic knee joint. To this end, connection features are regularly provided at the proximal end on the prosthetic foot in order to establish a stable and permanent connection with the proximal prosthetic component. Prosthetic feet are usually provided with a cosmetic covering, which consist of plastic and are embodied approximately in the form of a natural foot.

From the structural point of view, the simplest form of a prosthetic foot is a rigid foot. However, a rigid foot has significant disadvantages in view of the elastic properties or the rollover properties. More complex designs include dampening elements or heel springs for damping the momentum upon heel strike. It is likewise possible for a spring to be arranged in the forefoot region in order to enhance the rollover characteristics of the foot during the stance phase and to store and then release deformation energy so as to assist the prosthetic foot user when walking.

Opportunities exist for improvements in prosthetic feet designs to improve energy feedback, dampening, and other properties that increase performance and user comfort and stability.

US 2003/0191541 A1 discloses a prosthetic foot, comprising a base spring, a top spring assembly, a connector and a heel cushion. The base spring has a toe end portion and a heel end portion. The top spring assembly comprises a first spring member having a distal end and a proximal end; a second spring member having a distal end and a proximal end; a first connection provided between the distal ends of the first and second spring members, and a second connection provided between the distal end of the second spring member and a top surface of the base spring; and a slot extending rearward from the distal ends of the first and second spring members along at least half of a length of the first portion of the top spring assembly. The connector is connected to the proximal ends of the first and second spring members and configured to connect the prosthetic foot to a lower limb prosthetic. The heel cushion is mounted to the base spring at a location spaced forward of a heel end of the base spring, the heel cushion arranged to contact a bottom surface of the second spring member during use of the prosthetic foot.

US 2016/0081821 A1 relates to a prosthetic foot including a foot member and a heel member. A distal end of the heel member is coupled to the foot member. The foot member comprises a tongue portion defined by a slot in the foot element. The prosthetic foot further comprises a mechanism configured to operatively connect or disconnect the tongue protion from the remainder of the foot member. When the tongue portion is connected to the remainder of the foot member, the foot member exhibits greater stiffness.

US 6,241,776 B1 discloses a prosthetic foot including a forefoot member, a heel member and a forefoot reinforcement member. The forefoot reinforcement member has a base section coupled to the top side of the forefoot member. The forefoot reinforcement member distally extends above the forefoot member to a free section. The free section is moveable with respect to the forefoot member and operates within the range of motion of the forefoot member. The prosthetic foot also comprises a flexible strap coupled to the proximal ends of the forefoot member and the forefoot reinforcement member. The flexible strap transfers some of the heel load to the forefoot reinforcement member.

US 8 007 544 B2 is related to a low profile prosthetic with a foot member extending at an incline from an anterior portion to a posterior portion thereof and configured to flex during motion. An adapter is mounted solely at a posterior section thereof to the posterior portion of the foot member so that the adapter's anterior section can move relative to the foot member and "roll-up" onto the foot member during motion.

US 2012/0271434 A1 is related to a prosthetic foot with a forefoot spring, a heel spring and a base spring. The base spring is connected to the heel spring and to the forefoot spring. The base spring has receiving means for the forefoot spring and the heel spring, into which receiving means the heel spring and the forefoot spring can be inserted. The heel spring is connected to the forefoot Spring via a coupling element, and the coupling element extends forwards along the forefoot spring at least via one portion thereof.

US 2015/0374514 A1 is related to a prosthetic foot and a foot cover for providing a more natural appearance and improved performance. A foot element for a prosthetic foot can extend from a heel end to a toe end and include a heel, arch, forefoot, and toe regions. The forefoot region can be wider than the arch and heel regions. The toe region can include a U-shaped cut out to define a big toe.

WO 2000/027317 A1 is related to a resilient forefoot member having a base end coupled proximal an attachment location and extending forward to a toe end at a toe location. The forefoot member defining an arch section between the base end and the toe end, and having a range of motion with multiple stages of advancement including at least a normal range and an extreme range and having a resistance response to an applied force. The forefoot member has a forefoot reinforcement member having a base section coupled to the forefoot member at the arch section and extending to a free end at a location between the arch section and the attachment section and spaced from the forefoot member. The free end being disposed within the extreme range of motion of the forefoot member and having a range of motion within the extreme range of motion of the forefoot member, such that the forefoot reinforcement member influences the range of motion and resistance response of the forefoot member.

One aspect of the present disclosure relates to a prosthetic foot having a base spring, a top spring assembly, a connector assembly, and a heel cushion. The base spring has a toe end portion and a heel end portion. The top spring assembly includes first and second spring members, first and second connections, first and second portions, and a slot. The first spring member has a distal end and a proximal end. The second spring member extends substantially parallel with and spaced apart from the first spring member along substantially an entire length of the first spring member. The second spring member has a distal end and a proximal end. The first connection is provided between the distal ends of the first and second spring members. The second connection is provided between the distal end of the second spring member and a top surface of the base spring. The first portion is arranged at a first angle relative to a horizontal plane. The second portion extends from the first portion. The slot extends rearward from the distal ends of the first and second spring members along at least half of a length of the first portion of the top spring assembly. The connector may be releasably connected to the proximal ends of the first and second spring members, and configured to connect the prosthetic foot to a lower limb prosthesis. The heel cushion is mounted to the base spring at a location spaced forward of a heel end of the base spring. The heel cushion is arranged to contact a bottom surface of the second spring member during use of the prosthetic foot.

The first angle of the top spring assembly may be in the range of about 5° to about 30°. The second portion of the top spring assembly may extend from the first portion in a substantially vertical direction. The prosthetic foot may further include a spacer positioned between the proximal ends of the first and second spring members. The first bond connection may provide a spacing between the distal ends of the first and second spring members. The first bond connection may include an adhesive. The second spring member may be spaced apart from the first spring member by spacers positioned at the distal and proximal ends of the first and second spring members. A thickness of the first spring member may be within 15% of a thickness of the second spring member.

Another aspect of the present disclosure relates to a prosthetic foot having a connector, a base spring, a top spring assembly, and a heel cushion. The connector is configured to connect the prosthetic foot to a lower limb prosthesis. The base spring has a toe end portion and a heel end portion. The top spring assembly includes a first spring member having a distal end and a proximal end, a second spring member positioned between the base spring and the first spring member, the second spring member having a distal end and a proximal end, and first and second spacers. The first spacer is positioned between the distal ends of the first and second spring members. The second spacer is positioned between the proximal ends of the first and second spring members. The first and second spacers are configured to space the second spring member away from the first spring member when the prosthetic foot is in a rest state. During use of the prosthetic foot, a portion of the first spring member spaced between the distal and proximal ends of the first spring member moves relative to the second spring member. The top spring assembly further includes a distal end portion connected to the base spring and extending substantially horizontally, and a proximal end portion connected to the connector. The heel cushion is mounted to the base spring at a location spaced forward of a heel end of the base spring. The heel cushion is arranged to contact a bottom surface of the second spring member during use of the prosthetic foot.

The distal end portion of the top spring assembly may be connected to the base spring with a permanent bond connection, and material of the permanent bond connection may include an elastomeric material. The proximal end portion of the top spring assembly may include the proximal ends of the first and second spring members and may be releasably connected to the connector with at least one fastener. The distal ends of the first and second spring members may be connected to each other with a bond connection, and material of the bond connection may define the first spacer. The top spring assembly may have a first portion arranged at an angle in the range of about 5° to about 30° relative to a horizontal plane, and a second portion extending substantially vertically. The top spring assembly may be spaced apart from the base spring. The material of the bond connection may include an elastomeric material. The heel cushion may be releasably attached to the base spring, and the heel cushion may include an elastomeric material. The distal end of the second spring member may extend distally further than the distal end of the first spring member.

A further aspect of the present disclosure relates to a method of manufacturing a prosthetic foot. The method includes providing a base spring, a connector, a heel cushion, first and second spring members, and a spacer, arranging the first and second spring members in parallel with each other with portions of the second spring member positioned below and/or rearward of the first spring member, connecting distal ends of the first and second spring members to each other (e.g., by bonding using an elastomeric material), and arranging the spacer between proximal ends of the first and second spring members. The spacer spaces the first and second spring members apart when the prosthetic foot is in a rest position. The method also includes connecting the proximal ends of the first and second spring members to the connector with at least one fastener, connecting the distal end of the second spring member to a toe end portion of the base spring, the distal ends of the first and second spring members being arranged substantially parallel with a top surface of the base spring, and mounting the heel cushion to the base spring at a location spaced forward of a heel end of the base spring, the heel cushion contacting a bottom surface of the second spring member.

The proximal ends of the first and second spring members may be arranged substantially vertically. The method may also include providing the first and second spring members with a lower portion and an upper portion, arranging the lower portion at an angle of about 5° to about 30° relative to a horizontal plane, and arranging the upper portion substantially vertically. The base spring may include a sandal slot formed in the toe end portion, and bonding the distal end of the second spring member to the base spring may include connecting to the base spring at a location spaced posterior of the sandal slot. A portion of the first spring member may be movable into contact with the second spring member during use of the prosthetic foot. The connections between the first and second spring members, and between the top spring assembly and the base spring may be a bond connection using, for example, an elastomeric material. The material of the bond connections may space the first and second spring members from each other, and may space the top spring assembly from the base spring.

The foregoing has outlined rather broadly the features and technical advantages of examples according to the disclosure in order that the detailed description that follows may be better understood. Additional features and advantages will be described hereinafter. The conception and specific examples disclosed may be readily utilized as a basis for modifying or designing other structures for carrying out the same purposes of the present disclosure. Such equivalent constructions do not depart from the scope of the appended claims. Features which are believed to be characteristic of the concepts disclosed herein, both as to their organization and method of operation, together with associated advantages will be better understood from the following description when considered in connection with the accompanying figures. Each of the figures is provided for the purpose of illustration and description only, and not as a definition of the limits of the claims.

A further understanding of the nature and advantages of the embodiments may be realized by reference to the following drawings. In the appended figures, similar components or features may have the same reference label.
FIG. 1 is a front perspective view of an example prosthetic foot assembly in accordance with the present disclosure.
FIG. 2 is a rear perspective view of the prosthetic foot assembly of FIG. 1.
FIG. 3 is a front exploded perspective view of the prosthetic foot assembly of FIG. 1.
FIG. 4 is a rear exploded perspective view of the prosthetic foot assembly of FIG. 1.
FIG. 5 is a right side view of the prosthetic foot shown in FIG. 1.
FIG. 6 is a left side view of the prosthetic foot shown in FIG. 1.
FIG. 7 is a top view of the prosthetic foot shown in FIG. 1.
FIG. 8 is a bottom view of the prosthetic foot shown in FIG. 1.
FIG. 9 is a front view of the prosthetic foot shown in FIG. 1.
FIG. 10 is a rear view of the prosthetic foot shown in FIG. 1.
FIG. 11 is a cross-sectional view of the prosthetic foot shown in FIG. 7, taken along cross-section indicators 11-11.
FIG. 12 is a side view of another example prosthetic foot in accordance with the present disclosure.
FIG. 13 is a side view of another example prosthetic foot in accordance with the present disclosure.
FIG. 14 is a flow diagram illustrating an example method in accordance with the present disclosure.

While the embodiments described herein are susceptible to various modifications and alternative forms, specific embodiments have been shown by way of example in the drawings and will be described in detail herein. However, the exemplary embodiments described herein are not intended to be limited to the particular forms disclosed. Rather, the instant disclosure covers all modifications, equivalents, and alternatives falling within the scope of the appended claims.

The present disclosure is generally directed to prosthetic devices, and more particularly relates to prosthetic foot devices, which are also referred to as a foot prostheses. The prosthetic foot embodiments disclosed herein may provide certain advantages as compared to other prosthetic foot devices. For example, the prosthetic foot devices of the present application may provide improved rollover characteristics, such as increased smoothness during rollover. The prosthetic foot may include an upper or top spring assembly having a pair of leaf springs attached at opposite ends with a gap provided between the leaf springs. The top spring assembly is combined with a base spring and is attached to the base spring at a toe region of the base spring. A heel cushion is positioned between the top spring assembly and the base spring in a heel region of the base spring. The heel cushion may be mounted directed to the base spring and arranged to contact a bottom surface of the top spring assembly. The heel cushion may be interchangeable to adjust heel stiffness.

The toe end of the prosthetic foot may include a split extending along a length dimension of the prosthetic foot. The split may be arranged along a longitudinal centered line of the prosthetic foot to provide medial/lateral compliance. The split may extend through portions of the base spring and the top spring assembly.

The prosthetic foot design is targeted generally to meet the needs of a typical, active amputee. The prosthetic foot is designed for comfortable walking and many common conditions encountered by an amputee, rather than being narrowly focused towards a particular user group such as runners, skiers, swimmers, etc.

The top spring assembly may have various shapes and designs. In one example, the top spring assembly includes a generally horizontal portion that extends substantially parallel to the base spring, and an upper portion that extends in a substantially vertical direction to provide an L-shaped construction. Other designs have a lower profile that does not include the vertical portion of the L-shaped design. Still further embodiments may include a vertical portion having a greater length that extends further vertically upward relative to a base spring.

The base spring may extend further in an anterior direction than the most anterior point of the top spring assembly. The base spring may include a sandal slot position adjacent to the split formed along the centerline that provides medial/lateral compliance. In at least some embodiments, the first and second leaf springs of the top spring assembly may terminate at different anterior locations (e.g., the upper most leaf spring being spaced further posteriorly as compared to the lower leaf spring). Further, a heel end of the base spring may extend further posteriorly beyond the heel cushion. This arranged at the heel end of the base spring provides a heel lever arm located posterior to the heel cushion.

The prosthetic foot of the present disclosure may include various combinations of features that provide certain advantages as compared to known prosthetic feet. Once such combination of features includes a multi-spring top spring assembly wherein the springs are connected at opposite ends and include a gap or spacing between each other along the lengths of the springs between the connection points at the opposite ends. The top spring assembly is connected to a full-length base spring in a toe region of the base spring, and at a location that is spaced posterior of an anterior end of the base spring. The top spring assembly may be connected to the base spring at only one location. The prosthetic foot includes a replaceable elastomeric heel cushion positioned between the base spring and the top spring assembly in a heel end of the prosthetic foot. Typically, the heel cushion is detached from the top spring assembly, but is arranged to contact a bottom surface of the top spring assembly, particularly during use. The top spring assembly may include leaf springs having different lengths. The different lengths may allow a gradual change in the stiffness of the top spring assembly at various locations along the length of the prosthetic foot (e.g., in the toe region). One advantage of this particular combination of features is that the flexibility of the base plate does not have as much influence on performance. To some degree, the base plate may, at the connection point to the top spring assembly, pivot or otherwise move due to the type of connection that is provided between the top spring assembly and the base spring. In at least one example, the connection is provided by an adhesive bond or other bond structure that provides at least some flexibility and/or elasticity. Heel performance of the prosthetic foot may also be enhanced by this pivoting provided by a flexible adhesive bond between the base spring and the top spring assembly, or may be accomplished by flexibility in the base spring. In this particular embodiment, the prosthetic foot includes bond flexibility at the connection between the base spring and the top spring assembly as well as flexibility in the base spring provided by the structure of the base spring and the split in the toe end portion. A flexible bond between the upper spring assembly and the distal plate may act as a film hingeas described in U.S. Patent Publication No. 2017/0049584. A flexible distal plate may also contribute to film hinge behavior. It may be possible to tailor the contribution of the distal plate to heel performance by adjusting these and other design parameters. The heel performance is primarily determined by compressibility of the heel cushion, although the shape and position of the heel cushion may also contribute to heel performance. Thus, various features contribute to achieving a desired heel function for the prosthetic foot. By transferring load during heel strike from the posterior portion of the base spring to the posterior portion of the top spring assembly, the heel cushion reduces stress at the connection between the base spring and the upper spring assembly located in the anterior region of the prosthetic foot.

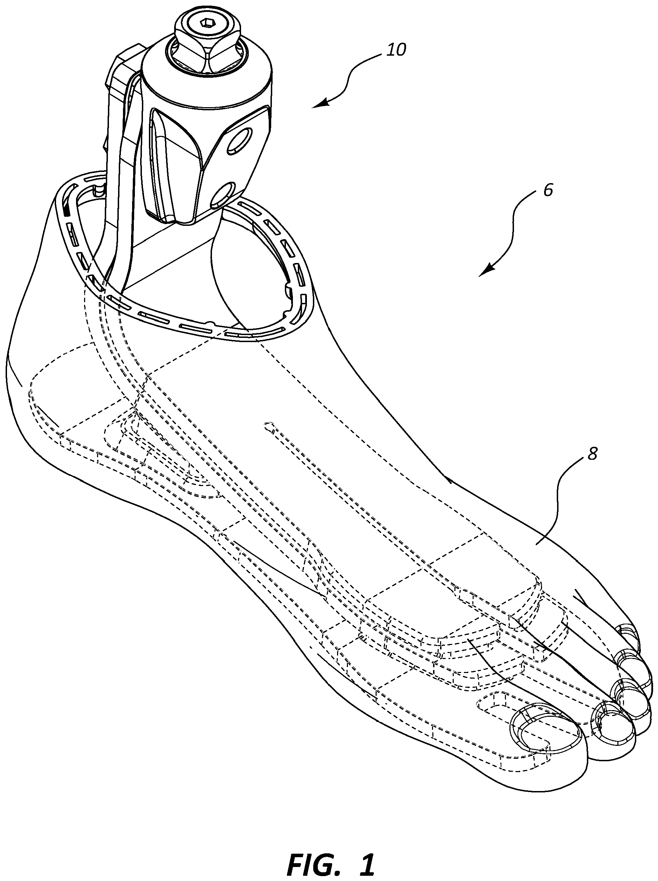

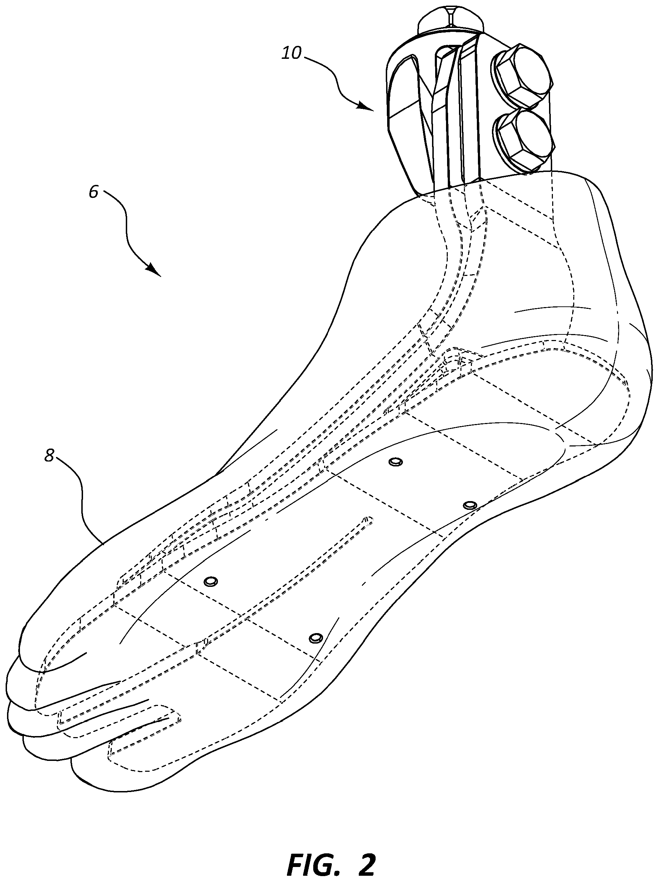

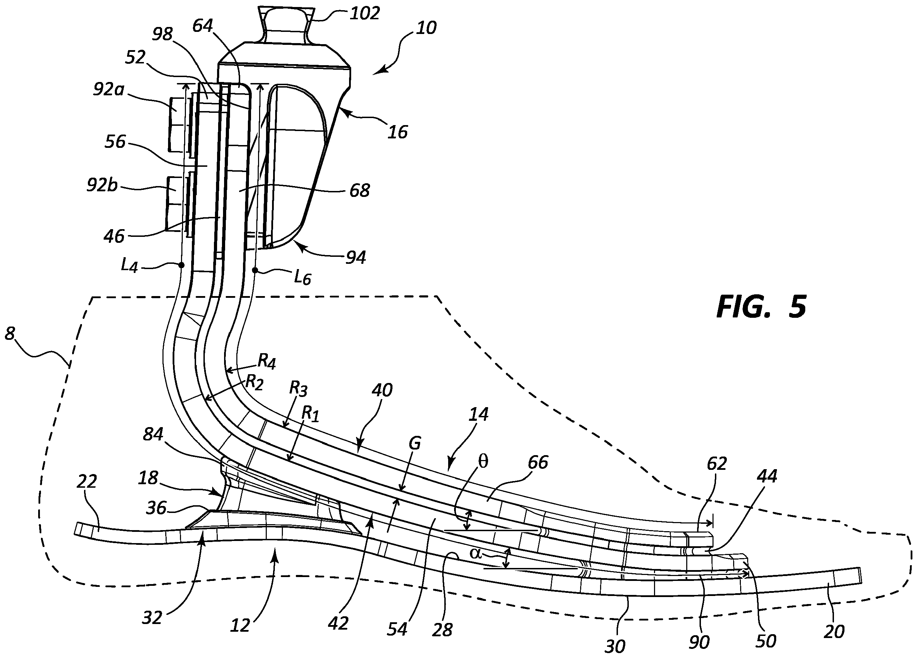

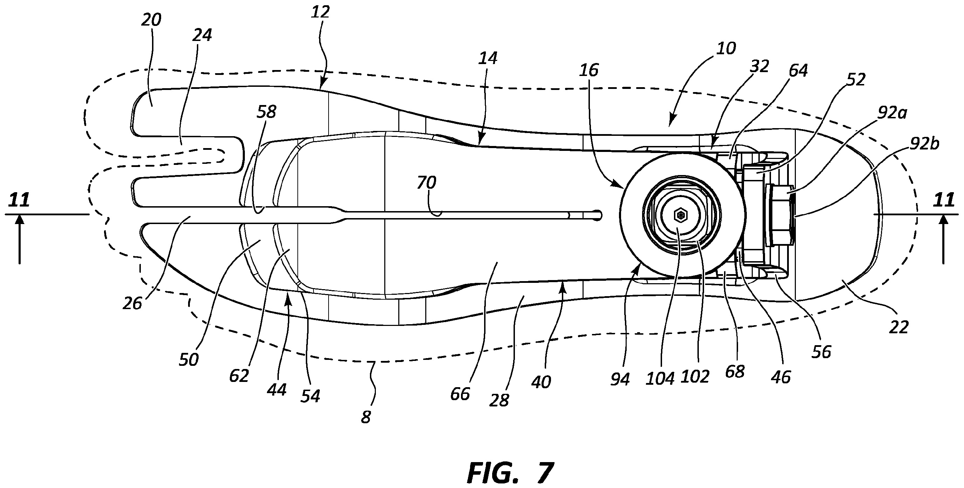

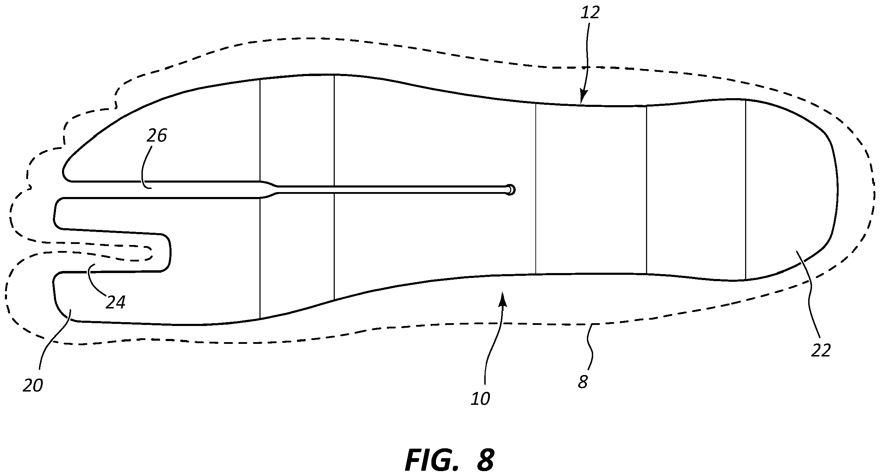

Referring now to FIGS. 1-4, an example prosthetic foot assembly 6 is shown and described. The prosthetic foot assembly 6 includes a cosmesis 8 and a prosthetic foot 10. The cosmesis 8 is a hollow structure in which portions of the prosthetic foot 10 are positioned. The cosmesis 8 provides an aesthetic covering for the prosthetic foot 10 to give the appearance of an actual foot. FIGS. 5-11 illustrate the prosthetic foot 10 in further detail. The prosthetic foot 10 is intended to be used inside a shoe, the heel height being raised to accommodate the heel of a typical shoe.

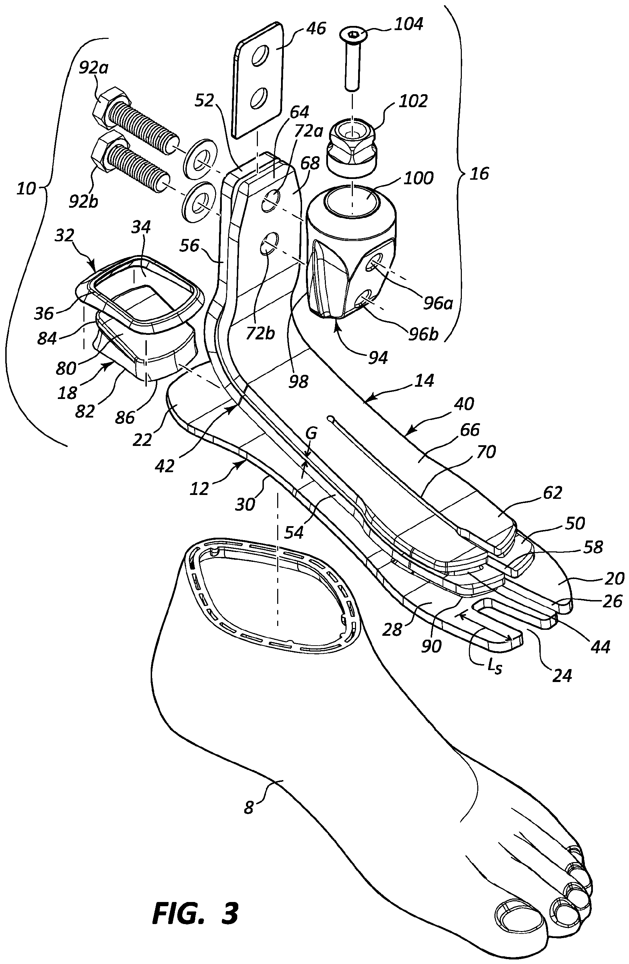

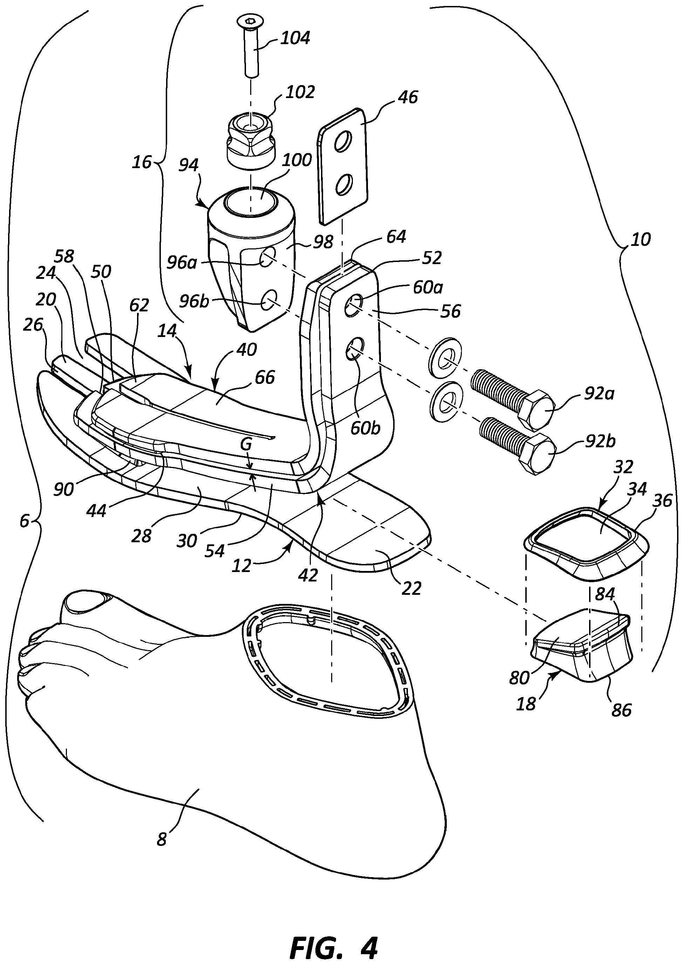

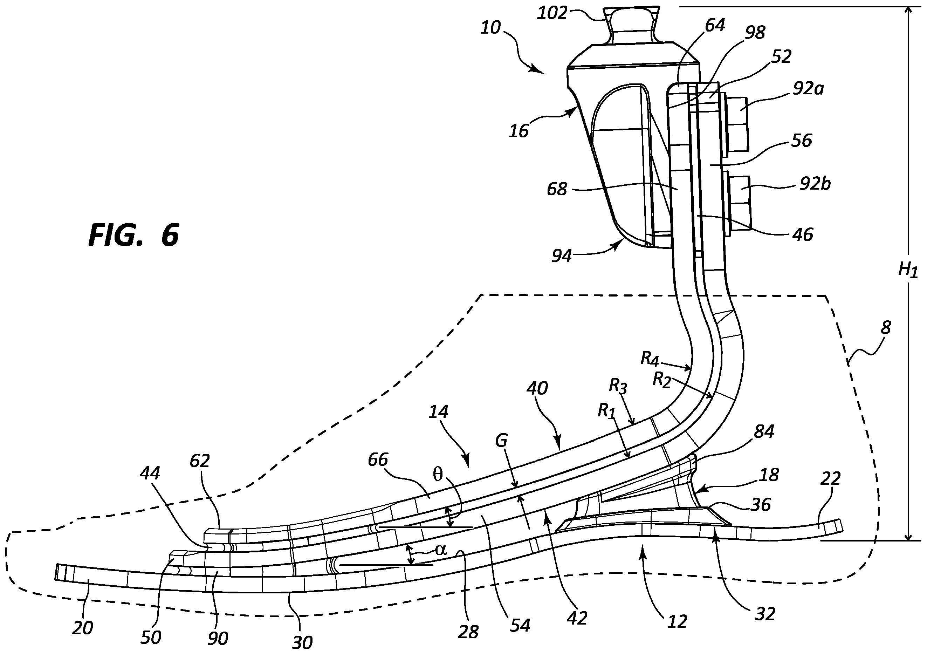

Referring now to FIGS. 3 and 4, the prosthetic foot 10 is shown including a base spring 12, a top spring assembly 14, a connector assembly 16, and a heel cushion 18. The top spring assembly 14 is connected to the base spring 12 in a toe end area at a toe end connection 90. The toe end connection 90 may include a bond connection formed by, for example, an adhesive bond. The toe end connection 90 may be formed using an elastic, flexible material that provides at least some relative movement between the base spring 12 and top spring assembly 14 (e.g., rotational movement about a vertical axis, compression, and translational movement in the anterior/posterior and/or medial/lateral direction). The toe end connection 90 may provide the sole connection point between the base spring 12 and top spring assembly 14. Typically, the heel cushion 18 is mounted directly to a top surface of the base spring 12 and arranged to contact a bottom surface of the top spring assembly 14 as shown in, for example, FIGS. 5 and 6. The heel cushion 18 may be releasably connected to the base spring 12. Alternatively, heel cushion 18 may be releasably connected to the top spring assembly. In at least some examples, the heel cushion 18 is connected to the base spring 12 with an interference fit connection using, for example, a retainer 32 that is mounted to the top surface of the base spring 12. The heel cushion 18 may be replaceable with other heel cushions having different properties such as increased or reduced stiffness, compressibility, damping capability, etc. Heel cushions of different sizes and shapes may also be used in place of the heel cushion 18 shown in the figures. In some examples, the prosthetic foot 10 may be operable without any heel cushion 18.

The connector assembly 16 may be releasably attached to the top spring assembly 14 at its proximal end. In at least one example, the connector assembly 16 is releasably connected using one or more fasteners 92a, 92b. Connector assemblies with different connector features such as a pyramid connector 102 may be used. In at least some examples, the pyramid connector is a replaceable component of the connector assembly 16. In other embodiments, the pyramid connector is integrally formed with remaining portions of the connector assembly and mounted directly to the top spring assembly. Other connector features besides a pyramid connector may be used as part of the connector assembly for securing the prosthetic foot 10 to another prosthetic member such as a lower leg pylon, a socket, or the like.

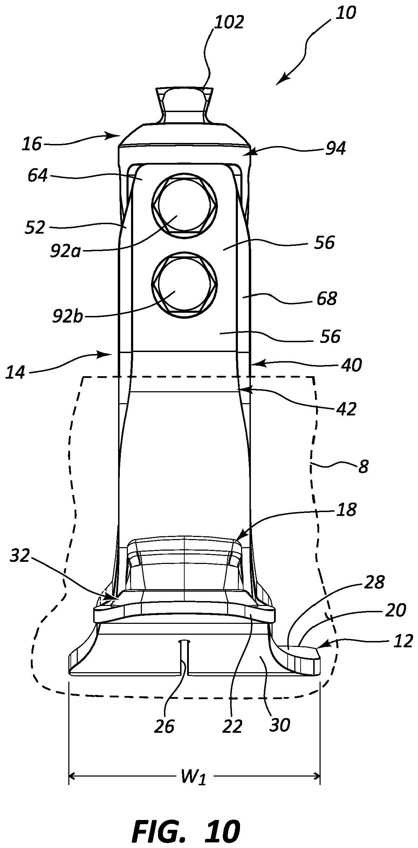

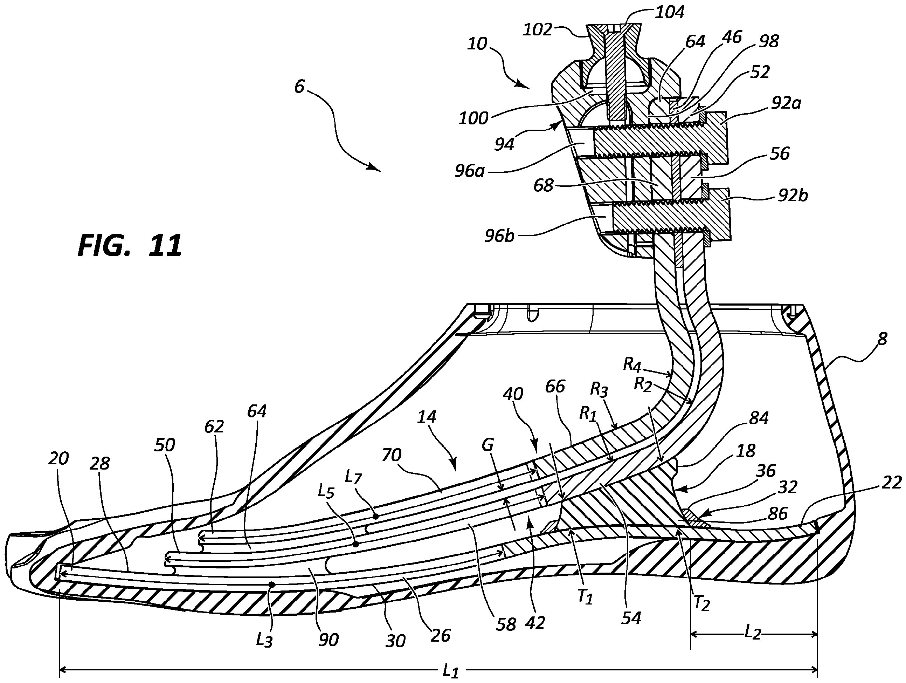

The base spring 12 is shown including a toe end 20, a heel end 22, a sandal slot 24, and a balance slot 26. The base spring 12 may also include a top surface 28, a bottom surface 30, and a heel cushion retainer 32 positioned at a heel end portion of the base spring 12. The retainer 32 may include a cavity 34 and a rim 36 to help releasably secure the heel cushion 18 to the base spring 12. The base spring 12 may also include a maximum length L₁ (*see* FIG. 11), a maximum width W₁ (*see* FIG. 10), a heel lever length L₂ (*see* FIG. 11) extending posterior of the heel cushion 18, and a balance slot length L₃ (*see* FIG. 11). Typically, the maximum length L₁ is in the range of about 17.78 to about 30.48 cm, depending on foot size. The maximum width W₁ is typically in the range of about 2.54 to about 7.62 cm. The heel lever length L₂ is typically in the range of about 2.54 to about 7.62 cm, and more particularly in the range of about 2.54 to about 5.08 cn. The L₃ is typically in the range of about 7.62 to about 20.32 cm, and more particularly about 12.7 to about 17.78 cm. The dimensions of the base spring 12 may depend largely on the foot size.

The sandal slot 24 may also have a length Lₛ. The length Lₛ is typically in the range of about 1.27 to about 5.08 cm. The sandal slot 24 is formed in the toe end portion of the base spring 12 and extends posterior from an interior most edge of the base spring 12. The balance slot 26 is also formed at the toe end portion beginning at the anterior most edge of the base spring 12 and extending posteriorly. In at least some embodiments, the balance slot 26 is aligned with a longitudinal center line of the base spring 12. The balance slot 26 may provide enhanced medial/lateral compliance for the prosthetic foot 10, particularly when walking on uneven surfaces.

As shown in at least FIGS. 5 and 6, the base spring 12 has a contoured shape along its length. The side profile of the base spring 12 undulates between concave and convex shapes. In some examples, the distal surface of the base spring 12 is preferably convex in an anterior section, transitions to concave in an arch or mid-section, and may transition back to convex at the posterior end. These contours and the location of the contours, particularly relative to the toe end connection 90 and the heel cushion 18, may provide improved rollover smoothness, enhanced energy feedback to the user, stability, and comfort during use of the prosthetic foot. Providing the lever portion extending posterior of the heel cushion 18 may also provide improved smoothness in the rollover and energy feedback during use.

The top spring assembly 14 is shown including first and second spring members 40, 42, a first spacer 44 at the toe end portion of the prosthetic foot, a second spacer 46 positioned at a proximal end of the top spring assembly 14 and a gap G provided between the first and second spring members 40, 42 along their entire length. The first and second spring members 40, 42 may be referred to as leaf springs. The first and second spring members 40, 42 may extend generally in parallel with each other along their entire lengths. The first spacer 44 may be provided as a bond connection between the first and second spring members 40, 42. In at least some examples, the first spacer 44 comprises the same bond material as used for the toe end connection 90 between the top spring assembly 14 and the base spring 12. In at least some embodiments, the first spacer 44 is positioned generally in alignment with the toe end connection 90 so as to be positioned vertically above the toe end connection 90, or at least partially overlapping the toe end connection 90 in a length dimension of the base spring 12. The first spacer 44 may provide a permanent connection between the first and second spring members 40, 42. The material of first spacer 44 may provide at least some relative movement between the first and second spring members 40, 42 (i.e., rotational movement about a vertical axis, translational movement in an anterior, posterior or medial/lateral direction, compression, etc.). The material of first spacer 44 may be elastic so as to return to its original shape upon removal of a force that is used to compress or deform the first spacer 44.

The second spacer 46 may comprise a rigid material that is non-compressible and/or non-elastic. The second spacer 46 may be positioned at a proximal most end of the top spring assembly 14. The second spacer 46 may be aligned with the connector assembly 16, or at least portions thereof. In the illustrated embodiment, the second spacer 46 includes apertures through which the fasteners 92a, 92b extend for connection of the connector assembly 16 to the top spring assembly 14.

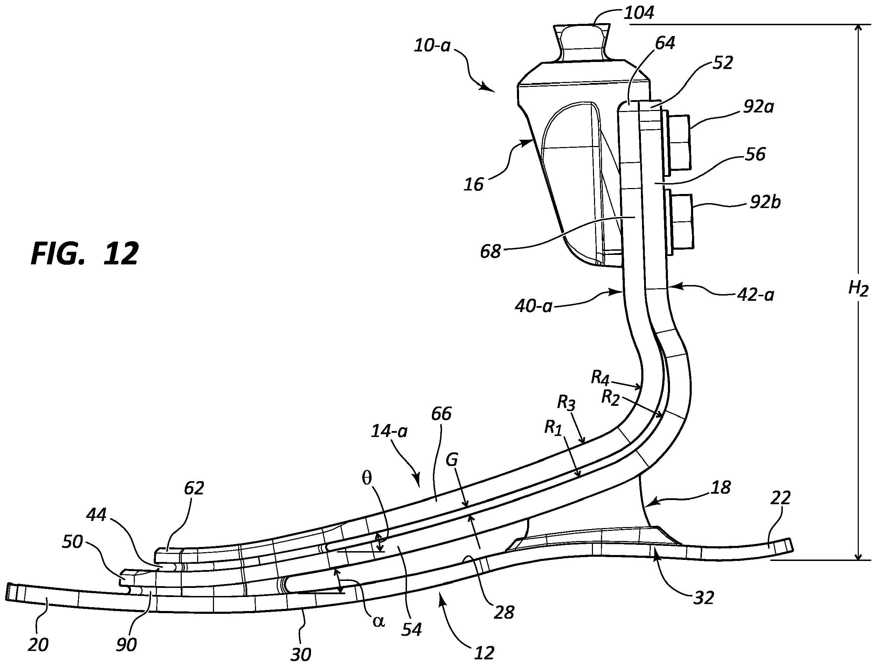

The first and second spacers 44, 46 may define the size of the gap G when the prosthetic foot 10 is in a rest state. Typically, the gap G is provided along an entire length of the first and second spring members 40, 42 when the prosthetic foot 10 is in a rest state (*i.e.,* prior to application of a force during use of the prosthetic foot 10). Alternatively, the two upper springs 40, 42 may abut (e.g., directly contact each other) at the connector location as shown in FIG. 12. The gap G may vary in size during operation of the prosthetic foot 10. For example, the gap G may reduce in size at the first spacer 44 if the material of the first spacer 44 is compressible during use. In another example, the gap G may reduce or change size at locations between the first and second spacers 44, 46 during use. For example, applying a force from a user during a gait cycle may change the size of gap G at various phases of the gait cycle (e.g., at heel strike, stance phase, and toe off), as the forces are applied and released during use by a wearer, those forces are absorbed and/or fed back through the base spring 12 and heel cushion 18. In at least some embodiments, the first spring member 40 may come into contact with the second spring member 42 during use of the prosthetic foot (*i.e.,* the gap reduces to zero).

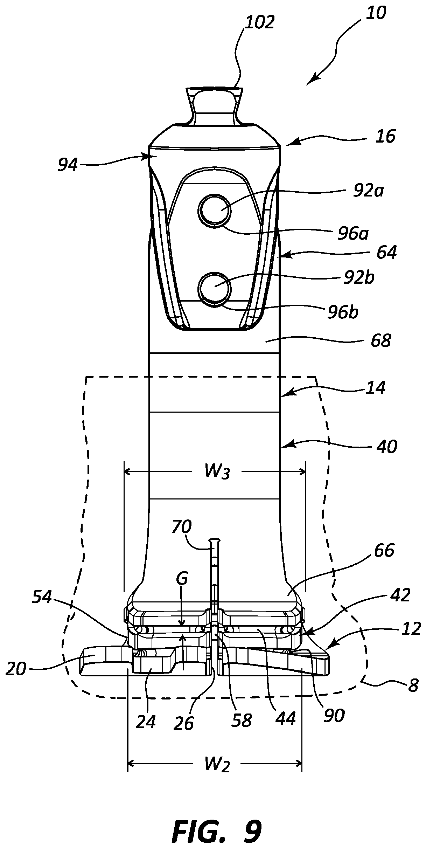

The second spring member 42 is shown having an anterior end 50, a proximal end 52, a horizontal portion 54, a vertical portion 56, a slot 58, and fastener apertures 68a, 60b. The second spring member 42 has an overall length L₄ as shown in FIG. 5, a maximum width W₂ as shown in FIG. 9, and a length L₅ for slot 58 as shown in FIG. 11. The horizontal portion 54 may have a radius of curvature R₁ as shown in FIGS. 5 and 6. The second spring member 42 also has a bend radius R₂ at the intersection between the horizontal and vertical portions 54, 56. The radius of curvature R₁ may be variable along the length from the anterior end 50 in a posterior direction. The radius R₁ is typically in the range of about 25.4 to about 76.2 cm, and more particularly about 45.72 to about 55.88 cm. The bend radius R₂ is typically in the range of about 1.905 to about 7.62 cm, and more particularly about 2.54 to about 3.81 cm. Although the horizontal portion 54 extends generally in a horizontal direction, it may be arranged at an angle α relative to a horizontal plane as shown in at least FIG. 5. The angle α may be measured along that part of the horizontal portion 54 that is posterior of the first spacer 44. The angle α is typically in range of about 2° to about 30°, and more particularly about 5° to about 15°.

The first spring member 40 may include an anterior end 62, a proximal end 64, a horizontal portion 66, a vertical portion 68, a slot 70, and fastener apertures 72a, 72b. The first spring member 40 may have an overall length L₆ as shown in FIG. 5, a maximum width W₃ as shown in FIG. 10, and a length L₇ for slot 70 as shown in FIG. 11. The slot 70 may be formed at the anterior end 62 and extend posteriorly. The slot 72 may be aligned with the slot 58 of the first spring member 40 and the balance slot 26 formed in base spring 12. In at least some examples, the slots 26, 58, 70 may extend in a posterior direction to a common location. The slots 26, 58, 70 may terminate at different locations in the anterior direction as shown in at least FIG. 3. The slots 58, 70 may be aligned with a center line of the base spring 12 and top spring assembly 14 so as to provide balanced medial/lateral pronation and compliance during use of the prosthetic foot.

The second spring member 42 may include a radius of curvature R₄ for the horizontal portion 66 along its length. The radius R₃ may be variable along its length from the anterior end 62 toward the vertical portion 68. The first spring member 40 may also include a bend radius R₄. Typically, the radiuses R₃, R₄ are similar to the radiuses R₁, R₂. Radius R₃ may be in the range of about 25.4 to about 76.2 cm, and more particularly about 45.72 to about 55.88 cm. R₄ may be in the range of about 1.905 to about 7.62 cm, and more particularly about 1.27 to about 2.54 cm. Because the first spring member 40 is positioned on an upper side and nested within the concave curvature of the second spring member 42, the radius R₄ is typically smaller than the radius R₂.

The horizontal portion 66 may be arranged at an angle θ relative to a horizontal plane as shown in FIG. 5. The angle θ may be in the range of about 2° to about 30°, and more particularly about 5° to about 20°. The angle θ typically is the same or similar to the angle α for horizontal portion 54.

The top spring assembly 14 is mounted to the base spring 12 as shown in at least FIGS. 5-11. The heel cushion 18 is arranged to contact a bottom or downward facing side or surface of the top spring assembly 14 (e.g., a bottom surface of second spring member 42 as shown in FIG. 5). Although the heel cushion 18 is shown connected to the base spring 12 and not the top spring assembly 14, other embodiments may provide the heel cushion 18 connected to both the base spring 12 and top spring assembly 14, or connected only to the top spring assembly 14 (e.g., the retainer 32 is mounted to the bottom surface of second spring member 42 for releasable attachment of the heel cushion 18).

The heel cushion 18 may be releasably mounted to the base spring 12 (or top spring assembly 14). Alternatively, the heel cushion 18 may be permanently connected to the base spring 12. The replaceability of heel cushion 18 may provide customization of the amount of cushioning, energy dampening, and the like provided by heel cushion 18. Heel cushion 18 may be connected with an interference fit connection. Other embodiments may provide for the heel cushion 18 to be secured with a positive connection such as, for example, a fastener, clip, bracket or the like.

The heel cushion 18 may include a top surface 80 *(see* FIGS. 3 and 4), a tapered shape having a variable thickness along its length, a bottom surface 82, and top and bottom perimeter rims 84, 86. The tapered shape may provide for a smaller thickness T₁ at an anterior end as compared to a greater thickness T₂ at a posterior end of the heel cushion 18, as shown in FIG. 11. The tapered shape of the heel cushion 18 may match the angle α and/or curvature R₁ of the second spring member 42. As such, the top surface 80 may have a contoured shape rather than a planar shape. Similarly, the bottom surface 82 may have a shape that matches the contour or curvature of the top surface of the base spring 12, as shown in at least FIG. 11.

The heel cushion 18 may comprise a shock absorbing, dampening material such as, for example, silicone or urethane elastomers including, for example, silicone or urethane foams. In some embodiments, the heel cushion 18 may include a plurality of different materials, layers of materials, or separate components that are secured together as an assembly to provide the desired cushioning properties. In one example, the heel cushion 18 includes a foam material encapsulated within a protective polymer shell. In another example, the heel cushion 18 includes a gel material or capsule that is encapsulated within a foam material.

The connector assembly 16 includes a base 94, fastener bores 96a, 96b, a spring assembly seat 98, a bore 100, a pyramid connector 102, and a fastener 104. The fastener bores 96a, 96b are receptive of the fasteners 92a, 92b. In at least some examples, the fastener bores 96a, 96b include threads that threadably engage with threads of the fasteners 92a, 92b. In other examples, the fastener bores 96a, 96b are pass-through bores and the fasteners 92a, 92b extend through the bores and threadably engage with nuts positioned on an opposite side of the base 94. The spring assembly seat 98 may be shaped and sized to interface with one or both of the first and second spring members 40, 42. The spring assembly seat 98 may include a lip or edge that engages a proximal most surface of, for example, the first spring member 40 at the proximal end 64. This lip or edge may provide a more secure connection and interface between the connector assembly 16 and the top spring assembly 14.

The bore 100 may be sized to receive a portion of the pyramid connector 102. The bore 100 may include a first portion sized to receive the pyramid connector 102 and a second portion (e.g., a threaded bore) that threadably engages with threads of the fastener 104. The bore 100 may be sized to receive pyramid connectors 102 of different sizes and shapes, or different types of connectors that may be used to secure the prosthetic foot 10 to a separate lower leg prosthetic member.

The base spring 12 and first and second spring members 40, 42 may comprise a fiber reinforced composite material such as, for example, carbon fiber reinforced composite. The first spacer 44 may include an adhesive bond comprising a flexible adhesive such as, for example, a urethane adhesive having a Shore A hardness in the range of about 70 to about 95. During manufacture of the top spring assembly 14, the first and second spring members 40, 42 may be bonded together using a removable gasket between the springs to create a sealed space for the adhesive, and the adhesive is then injected into the space.

The first spring member 40 may be shorter in length Ls than the length L₄ for the second spring member 42. This difference in length may allow for a somewhat gradual change in stiffness in the top spring assembly 14. Although two spring members 40, 42 are shown as part of the top spring assembly 14, other embodiments may utilize more than two leaf spring elements, and the leaf spring elements may have the same or different lengths.

The second spacer 46 may comprise a lightweight material such as, for example, aluminum, nylon or fiberglass sheet material (e.g., fiberglass G-10). The top spring assembly 14 may provide a connection between the first and second spring members 40, 42 at opposite ends with a gap G provided there between, thereby providing a number of unexpected structural advantages. These advantages in connection with the type of spacers 44, 46, the toe end connection 90, the heel cushion 18, and/or other features may provide a number of performance advantages as compared to known prosthetic feet. For example, a dual, narrow cantilever beam, one located above the other and with a space in between the upper and lower beams, and with frictionless spacer at the free end to transmit an applied vertical force from the upper beam to the lower beam at the free end, may result in about 15-25% reduction in bending stress and about 30-45% reduction in shear stress as compared to an equivalent stiffness single cantilever beam. While the top spring assembly 14 does not duplicate the boundary conditions of this idealized model exactly, these boundary conditions are predominately accurate. Because stresses are reduced by using the dual upper spring design, a prosthetic foot utilizing this dual spring design exhibits at least one of improved durability and improved flexibility as compared to single spring designs and dual spring designs without at least one spacer included. Many of the advantages of the dual cantilever beam designs disclosed herein may be maximized when the two beams (e.g., first and second spring members 40, 42) have substantially equal bending stiffness. If the beams are constructed of unidirectional fiber reinforced composite lamina, the maximum strength/stiffness ratio may be best achieved when both beams have substantially the same lamina orientation and thickness. As the difference between the bending stiffness of the upper and lower beams increases, the advantages of a dual cantilever spring design typically diminish.

The heel cushion 18 may comprise a silicone or urethane elastomer (e.g., an elastomer with the Shore hardness range of about 50A to about 90A). The heel cushion 18 may be retained with retainer 32 in a way that extends around an entire perimeter of the heel cushion 18. Other embodiments may provide for a retainer that extends around only a portion of perimeter of the heel cushion 18. The retainer 32 may be bonded to the top surface 28 of the base spring 12 using, for example, an adhesive. In some embodiments, both the adhesive and the retainer 32 are somewhat flexible to avoid detachment of the retainer 32 from the base spring 12 when the base spring 12 flexes during use. The retainer 32 and adhesive may comprise a plastic material having a Shore hardness in the range of, for example, about 90A to about 50D. Alternatively, the retainer 32 may be cast into the structure of base spring 12 along the top surface 28 thereof, which may eliminate the need for use of an adhesive or other bonding agent.

The retainer 32 may help keep the heel cushion 18 in place by utilizing geometric interlocking features. These interlocking features may include angled (*e.g.,* wedge-shaped) features in the retainer and along an exterior of the heel cushion 18, wherein corresponding surfaces interface to provide a connection The heel cushion 18 may be deformed or compressed in order to fit into the interior of the retainer 32, and then expanded automatically to its original shape thereby creating an interference fit connection between the features of the retainer 32 and the heel cushion 18. Alternatively, the retainer 32 and the heel cushion utilize a rib that fits into a recess, wherein the rib and recess may be formed on either the retainer 32 or heel cushion 18.

Generally, the base spring 12 extends from the toe region to a heel region of the prosthetic foot. The base spring 12 may extend from an interior most point of the prosthetic foot to a posterior most point of the prosthetic foot. The top spring assembly 14 may be connected to the base spring 12A at a location spaced anterior of an anterior most edge of the base spring 12. In at least one example, the top spring assembly 14 is positioned posterior of the sandal slot 24 formed at the distal end of the base spring 12. The base spring 12 may extend in an anterior direction at least as far as an anterior most point along a length of the top spring assembly 14.

As discussed above, the slot or split 26 formed in the base spring 12 from the anterior edge in a posterior direction may be aligned with the slots or slits 58, 70 formed in the top spring assembly 14 from the anterior end of the top spring assembly 14 extending in a posterior direction. These slots or splits may provide for the entire prosthetic foot 10 to be divided into medial and lateral sides at least in the toe and midfoot regions of the prosthetic foot.

The top spring assembly 14 includes first and second spring members 40, 42 that extend to different anterior positions along the length of the prosthetic foot. At least FIG. 5 illustrates the second spring member 42 extending further in an anterior direction than the first spring member 40. The first spacer 44 is positioned at the anterior most edge of the second spring member and spaced posterior of the anterior most edge of the second spring member 42.

The top spring assembly 14 extends generally parallel with the base spring 12 in the toe and midfoot regions of the base spring 12. The top spring assembly 14 may extend in a generally vertical direction relative to the base spring 12 in the heel end portion of the prosthetic foot. As described above, other embodiments may provide for the top spring assembly 14 to continue extending in a generally horizontal or slightly angled direction relative to the base spring 12 and/or a horizontal plane through the heel end portion.

Referring now to FIG. 12, another example prosthetic foot 10-a is shown. The prosthetic foot 10-a includes the same or similar components as the prosthetic foot 10 described above with reference to FIGS. 1-11 with exception of the top spring assembly 14-a. The top spring assembly 14-a does not include second spacer 46 at its proximal end. The first and second spring members 40-a, 42-a of the top spring assembly 14-a are arranged in direct contact with each other at the proximal end. The elimination of spacer 46 may result in several changes in the top spring assembly 14-a. For example, the radius of curvature R₂ of the second spring member 42-a may be smaller than the radius of curvature R₂ of second spring member 42 shown in FIGS. 1-11. The radius of curvature R₄ of the first spring member 40-a may be greater than the radius of curvature R₄ of first spring member 40 shown in FIGS. 1-11. Removal of the spacer 46 may also alter the size of radius of curvatures R₁ and R₃ as compared to those values for first and second spring members 40, 42 shown in FIGS. 1-11 (*e.g.,* reduce R₁ and increase R₃).

Additionally, the gap G provided between the first and second spring members 40-a, 42-a may vary along the length of the first and second spring members 40-a, 42-a from the first spacer 44 toward the proximal end of top spring assembly 14-a where the first and second spring members 40-a, 42-a are in direct contact with each other. The first and second spring members 40-a, 42-a may extend generally in parallel with each other along the portions 54, 66 to provide a substantially constant gap G in that portion of the top spring assembly 14-a, have a greater or smaller gap G in the area of radius of curvatures R₂, R₄, and then have a tapered gap G to the point of contact between the end portions 56, 68.

The gap G may extending along only a portion of the length of the first and/or second spring members 40-a, 42-a. For example, the gap G may be provided along a percentage of the length L₄ of the second spring member 42-a, such as in the range of about 60% to about 90% of the length L₄. During use of the prosthetic foot 10-a, portions of the first and second spring members 40-a, 42-a may move toward and/or away from each other to alter the size of gap G at various locations along the length of the top spring assembly 14-a. In at least some embodiments, portions of the first and second spring members 40-a, 42-a may contact each other at locations spaced distal of where the first and second spring members 40-a, 42-a are shown contacting each other in FIG. 12.

The first spacer 44 may be provided as a bond connection between the first and second spring members 40-a, 42-a. In at least some examples, the first spacer 44 may comprise a relatively incompressible material such as those described above related to spacer 46. The first spacer 44 may be provided as a separate piece that is secured in place between the first and second spring members 40-a, 42-a using an adhesive or other bonding agent. The material of first spacer 44 may provide at least some relative movement between the first and second spring members 40-a, 42-a (i.e., rotational movement about a vertical axis, shear displacement or translational movement in an anterior, posterior or medial/lateral direction, compression, etc.). The material of first spacer 44 may be elastic so as to return to its original shape upon removal of a force that is used to compress or deform the first spacer 44.

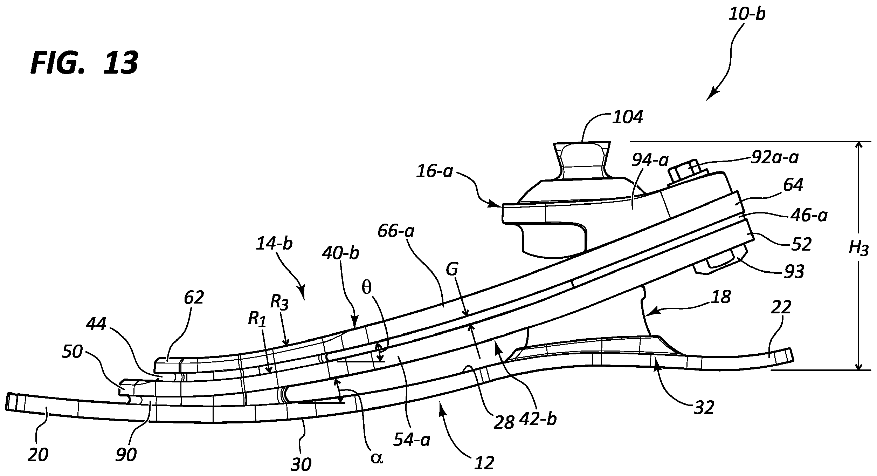

Referring now to FIG. 13, another example prosthetic foot 10-b is shown. The prosthetic foot 10-b includes many of the same or similar components as the prosthetic foot 10 and the prosthetic foot 10-a described above with reference to FIGS. 1-12 with exception of the top spring assembly 14-b. The top spring assembly 14-b does not include vertical portions 56, 68 as do the top spring assemblies 14 and 14-a. The first and second spring members 40-b, 42-b of the top spring assembly 14-b are arranged substantially horizontally along their length from their anterior ends 50, 62 to their proximal ends 52, 64. The elimination of vertical portions 56, 68 from the first and second spring members 40-b, 42-b may result in several changes in the top spring assembly 14-b as compared to the embodiments shown in FIGS. 1-12. For example, the horizontal portion 54-a, 66-a of the first and second spring members 40-b, 42-b may be longer than the horizontal portions 54, 66a of the first and second spring members 40, 42 shown in FIGS. 1-12. The horizontal portions 54-a, 66-a may extend from the anterior ends 50, 62 to the proximal ends 52, 64.

Additionally, the gap G provided between the first and second spring members 40-b, 42-b may have a reduced length from the first spacer 44 to the second spacer 46. The gap G may be substantially constant when the prosthetic foot 10-b is in a rest or unloaded state. During use of the prosthetic foot 10-b, portions of the first and second spring members 40-b, 42-b may move toward and/or away from each other to alter the size of gap G at various locations along the length of the top spring assembly 14-b. In at least some embodiments, portions of the first and second spring members 40-b, 42-b may contact each other.

The first spacer 44 may be provided as a bond connection between the first and second spring members 40-b, 42-b. In at least some examples, the first spacer 44 may comprise a relatively incompressible material such as those described above related to spacer 46. The first spacer 44 may be provided as a separate piece that is secured in place between the first and second spring members 40-b, 42-b using an adhesive or other bonding agent. In other examples, material of first spacer 44 may provide at least some relative movement between the first and second spring members 40-b, 42-b (*i.e.,* rotational movement about a vertical axis, shear displacement or translational movement in an anterior, posterior or medial/lateral direction, compression, etc.). The material of first spacer 44 may be deformable and/or elastic so as to return to its original shape upon removal of a force that is used to compress or deform the first spacer 44.

The second spacer 46-a may have many of the same or similar features and properties of second spacer 46 described above. The second spacer 46-a may have a different arrangement of holes to receive one or more fasteners 92a-a. In some example, the prosthetic foot 10-b may include a plurality of fasteners 92a-a to secure connector assembly 16-a to the top spring assembly 14. A base 94-a of the connector assembly 16-a may include one or more fastener holes to receive the one or more fasteners 92a-a. The base 94-a may be sized and arranged to orient the pyramid connector 102 in a vertical orientation when the prosthetic foot is unloaded, as shown in FIG. 13.

The fasteners 92a-a may be arranged side-by-side in a medial/lateral direction. In other arrangements, the fasteners 92a-a may be arranged in alignment with a length dimension of the prosthetic foot 10-b. Although only one fastener 92a-a is shown in FIG. 13, a plurality of fasteners 92a-a may be used. The fasteners 92a-a may provide a positive connection between the first and second spring members 40b, 42-b, a positive connection between the top spring assembly 14-b and the connector assembly 16-a, and/or a positive connection between one or both of the first and second spring members 40-b, 42-b and the spacer 46-a. In some examples, the fasteners 92a-a are connected directly to one or both of the first and second spring members 40-b, 42-b (e.g., to a threaded seat formed in one or both of the first and second spring members 40-b, 42-b), or may be connected to a nut 93 positioned on an opposite side of the top spring assembly 14-b, as shown in FIG. 13.

As is possible with the prosthetic feet 10, 10-a, the heel cushion 18 may be positioned between the base spring 12 and the top spring assembly 14-b. In at least some embodiments, the heel cushion 18 is mounted to the base spring 12, and may be releasably mounted to the base spring 12. The heel cushion 18 is arranged to contact a bottom surface of the top spring assembly 14-b, such as a long a bottom surface of the second spring member 42-b.

An overall height H₃ of the prosthetic foot 10-b may be substantially smaller than the heights H₁ and H₂ of the prosthetic feet 10, 10-a, respectively. The smaller height H₃ may provide a relatively low profile for the prosthetic foot 10-b and the prosthetic foot 10-b may be referred to as a low profile prosthetic foot. The prosthetic feet 10, 10-a may be referred to as high profile or medium profile prosthetic feet.

The prosthetic foot 10-b may provide many of the same or similar functions and benefits related to performance and user comfort as described herein related to prosthetic feet 10, 10-a. For example, the prosthetic foot 10-b may provide energy feedback, stability, force dampening and the like associated with the use of spaced apart spring members in the top spring assembly 14-b, the use of a heel cushion 18 arranged in the specific location and having the size and shape shown in FIG. 13, the shape and size of the top spring assembly 14-b and base spring 12, and the size, shape and orientation of the connector assembly 16-a. Furthermore, the base spring 12 and top spring assembly 14-b may include slots (e.g., slot 26 for base spring 12 and slots 58, 70 for first and second spring members 40-b, 42-b) that provide medial/lateral pronation and ambulation for the prosthetic foot 10-b, which may provide improved stability for the user, particularly on uneven ground surfaces.

The prosthetic feet 10, 10-a, 10-b may be referred as dual or multiple toe spring prosthetic feet. The prosthetic feet 10, 10-a, 10-b may be referred to as a single toe spring prosthetic feet. The heel assemblies, attachment assemblies, connection assemblies, and other features disclosed with reference to any single embodiment disclosed herein may be interchangeable with features of other prosthetic foot embodiments disclosed herein.

In alternative embodiments, the connection between the base spring and the top spring assembly and between the first and second spring members in the anterior region of the foot and may be provided with bolts or other fasteners. A rigid spacer may be provided between the spring members and/or between the top spring assembly and the base spring. The use of bolts or other fasteners in combination with an altered geometry of the first and second spring members may eliminate gaps that may otherwise exist at connection points at the anterior end of the prosthetic foot. In another embodiment, a connection between the first and second spring members may be made by wrapping carbon fiber or glass fiber around the first and second spring members at the connection point between the first and second spring members, and securing the spring members and the fiber by impregnating the fiber with epoxy or similar thermosetting resin. A similar connection may be made between the top spring assembly and the base spring.

In another example, the connection at the proximal end of the top spring assembly may be created by altering a geometry of the first and second spring members such that no gap exists at the connection points between the first and second springs (e.g., as shown in FIG. 12). In this arrangement, a gap may still be provided between the first and second spring members at other locations along their lengths (e.g., as shown in FIG. 12). In some embodiments, one or more of the first and second spring members may be inserted into a slot formed in the prosthetic connector (e.g., base 94 of connector assembly 16), and the first and second spring members are secured together and to the prosthetic connector with an adhesive or a fastener.

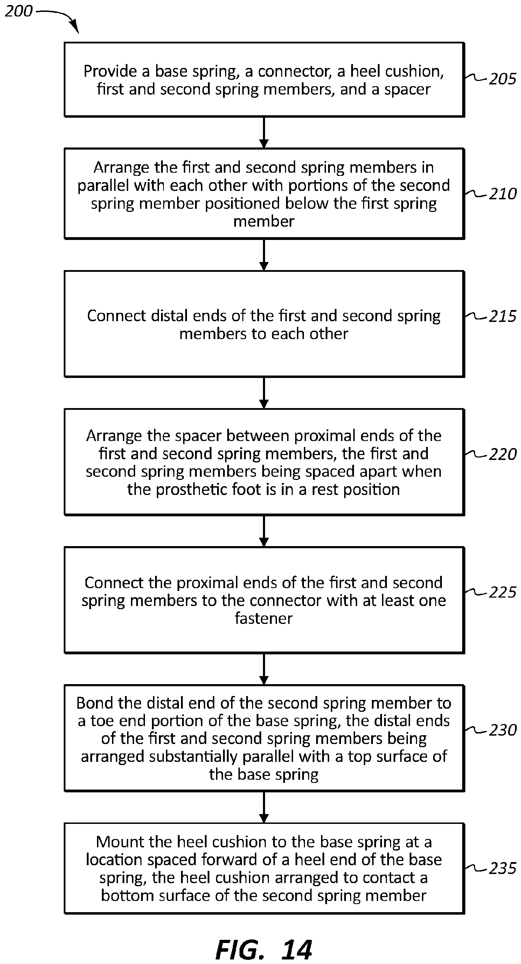

Referring now to FIG. 14, an example method 200 of manufacturing a prosthetic foot in accordance with the present disclosure is shown as a flow diagram. The method 200 includes, at block 205, providing a base spring, a connector, a heel cushion, first and second spring members, and at least one spacer. Block 210 includes arranging the first and second spring members in parallel with each other with portions of the second spring member positioned below the first spring member. The second spring member may also be positioned rearward of the first spring member. The method 200 includes, at block 215, connecting distal ends of the first and second spring members to each other, such as bonding together with an elastomeric material. The first and second spring members may be spaced apart from each other at their distal or anterior ends. Block 220 includes arranging the spacer between proximal ends of the first and second spring members. The spacer spaces the first and second members apart when the prosthetic foot is in a rest position. The method 200 includes, at block 225, connecting the proximal ends of the first and second spring members to each other and to the connector with at least one fastener. In some arrangements, the proximal ends of the first and second spring members are arranged substantially vertically. Block 230 includes connecting the distal ends of the second spring member to a toe end portion of the base spring, such as a bond connection using an adhesive. The distal ends of the first and second spring members may be arranged substantially parallel with a top surface of the base spring. Block 235 includes mounting the heel cushion to the base spring at a location spaced forward of a posterior end of the base spring, the heel cushion arranged to contact a bottom surface of the second spring member.

The method 200 may also include providing the first and second spring members with a lower portion and an upper portion, arranging the lower portion at an angle of about 5° to about 30° relative to a horizontal plane, and arranging the upper portion substantially vertically. The method 200 may include providing the base spring with a sandal slot formed in the toe end portion, and connecting the distal end of the second spring member to the base spring at a location spaced posterior of the sandal slot. The method 200 may include moving a portion of the first spring member into contact with the second spring member during use of the prosthetic foot.

The method 200 may be modified or altered in accordance with the present disclosure to include more or fewer steps than those illustrated in the figures. Accordingly, the flow diagram shown in FIG. 14 could include any step or variation of methods, features and/or functionality described herein. Other types of methods are possible in accordance with the present disclosure including, for example, methods of using a prosthetic foot, methods of storing and releasing energy in a prosthetic foot during use, and methods related to adapting or customizing performance of a prosthetic foot (e.g., by interchanging a heel cushion, a top spring assembly, or connection materials, such as the first spacer 44 or toe end connection 92).

The foregoing description, for purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the present systems and methods and their practical applications, to thereby enable others skilled in the art to best utilize the present systems and methods and various embodiments with various modifications as may be suited to the particular use contemplated.

Unless otherwise noted, the terms "a" or "an," as used in the specification and claims, are to be construed as meaning "at least one of." In addition, for ease of use, the words "including" and "having," as used in the specification and claims, are interchangeable with and have the same meaning as the word "comprising." In addition, the term "based on" as used in the specification and the claims is to be construed as meaning "based at least upon."

## Claims

1. A prosthetic foot, comprising:
a base spring (12) having a toe end portion and a heel end portion;
a top spring assembly (14), comprising:
a first spring member (40) having a distal end and a proximal end;
a second spring member (42) having a distal end and a proximal end;
a first connection provided between the distal ends of the first and second spring members (40, 42), and a second connection provided between the distal end of the second spring member (42) and a top surface of the base spring (12);
a slot extending rearward from the distal ends of the first and second spring members (40, 42) along at least half of a length of the first portion (54, 66) of the top spring assembly (14);
a connector (16) connected to the proximal ends of the first and second spring members (40, 42), and configured to connect the prosthetic foot to a lower limb prosthesis;
a heel cushion (18) mounted to the base spring (12) at a location spaced forward of a heel end of the base spring (12), the heel cushion (18) arranged to contact a bottom surface of the second spring member (42) during use of the prosthetic foot, **characterized in that**
the second spring member (42) extends substantially parallel with and spaced apart from the first spring member (40) along substantially an entire length of the first spring member (40);
a first portion (54, 66) of the first and second spring members (40, 42) is arranged at a first angle relative to a horizontal plane; and
a second portion (56, 68) of the first and second spring members (40, 42) extends from the first portion (54, 66).

2. The prosthetic foot of claim 1, **characterized in that** the first connection is provided by an adhesive.

3. The prosthetic foot of claim 1, **characterized in that** the second spring member (42) is spaced apart from the first spring member (40) by spacers (44, 46) positioned at the distal and proximal ends of the first and second spring members (40, 42).

4. The prosthetic foot of claim 1, **characterized in that** a thickness of the first spring member (40) is within 15% of a thickness of the second spring member (42).

5. A method of manufacturing a prosthetic foot according to claim 1, comprising:
providing a base spring (12), a connector, a heel cushion (18), first and second spring members (40, 42), and a spacer (46);
arranging the first and second spring members (40, 42) with portions of the second spring member (42) positioned below the first spring member (40);
connecting distal ends of the first and second spring members (40, 42) to each other;
arranging the spacer (46) between proximal ends of the first and second spring members (40, 42), the first and second spring members (40, 42) being spaced apart when the prosthetic foot is in a rest position;
connecting the proximal ends of the first and second spring members (40, 42) to the connector (16) with at least one fastener;
connect the distal end of the second spring member (42) to a toe end portion of the base spring (12), the distal ends of the first and second spring members (40, 42) being arranged substantially parallel with a top surface of the base spring (12);
mounting the heel cushion (18) to the base spring (12) at a location spaced forward of a heel end of the base spring (12), the heel cushion (18) arranged to contact a bottom surface of the second spring member (42), **characterized in that** the method further comprises:
arranging the first and second spring members (40, 42) in parallel with each other.

6. The method of claim 5, **characterized in that** it further comprises:
providing the first and second spring members (40, 42) with a lower portion (54, 66) and an upper portion (56, 68);
arranging the lower portion (54, 66) at an angle of about 5° to about 30° relative to a horizontal plane;
arranging the upper portion (56, 68) substantially vertically.

7. The method of claim 5, **characterized in that** the base spring (12) includes a sandal slot formed in the toe end portion, and connecting the distal end of the second spring member (42) to the base spring (12) includes bonding to the base spring (12) at a location spaced posterior of the sandal slot.

8. The method of claim 5, **characterized in that** a portion of the first spring member (40) is movable into contact with the second spring member (42) during use of the prosthetic foot.

## Patentansprüche

1. Prothesenfuß, umfassend:
eine Basisfeder (12) mit einem Zehenendabschnitt und einem Fersenendabschnitt;
eine obere Federanordnung (14), bestehend aus:
ein erstes Federelement (40) mit einem distalen Ende und einem proximalen Ende;
ein zweites Federelement (42) mit einem distalen Ende und einem proximalen Ende;
eine erste Verbindung, die zwischen den distalen Enden des ersten und zweiten Federelements (40, 42) vorgesehen ist, und eine zweite Verbindung, die zwischen dem distalen Ende des zweiten Federelements (42) und einer oberen Fläche der Basisfeder (12) vorgesehen ist;
einen Schlitz, der sich von den distalen Enden der ersten und zweiten Federelemente (40, 42) entlang mindestens der Hälfte einer Länge des ersten Abschnitts (54, 66) der oberen Federanordnung (14) nach hinten erstreckt;
ein Verbindungsstück (16), das mit den proximalen Enden der ersten und zweiten Federelemente (40, 42) verbunden ist und so konfiguriert ist, dass es den Prothesenfuß mit einer Prothese der unteren Gliedmaßen verbindet;
ein Fersenkissen (18), das an der Basisfeder (12) an einer Stelle angebracht ist, die vor einem Fersenende der Basisfeder (12) beabstandet ist, wobei das Fersenkissen (18) so angeordnet ist, dass es eine Bodenfläche des zweiten Federelements (42) während der Verwendung des Prothesenfußes berührt, **dadurch gekennzeichnet, dass**
sich das zweite Federelement (42) im Wesentlichen parallel zu und beabstandet von dem ersten Federelement (40) entlang im Wesentlichen einer gesamten Länge des ersten Federelements (40) erstreckt;
ein erster Abschnitt (54, 66) des ersten und zweiten Federelements (40, 42) in einem ersten Winkel relativ zu einer horizontalen Ebene angeordnet ist; und
ein zweiter Abschnitt (56, 68) des ersten und zweiten Federelements (40, 42) sich von dem ersten Abschnitt (54, 66) erstreckt.

2. Prothesenfuß nach Anspruch 1, **dadurch gekennzeichnet, dass** die erste Verbindung durch einen Klebstoff bereitgestellt ist.

3. Prothesenfuß nach Anspruch 1, **dadurch gekennzeichnet, dass** das zweite Federelement (42) durch Abstandshalter (44, 46), die am distalen und proximalen Ende des ersten und zweiten Federelements (40, 42) angeordnet sind, von dem ersten Federelement (40) beabstandet ist.

4. Prothesenfuß nach Anspruch 1, **dadurch gekennzeichnet, dass** eine Dicke des ersten Federelements (40) innerhalb von 15% der Dicke des zweiten Federelements (42) liegt.

5. Verfahren zur Herstellung eines Prothesenfußes nach Anspruch 1, umfassend:
eine Basisfeder (12), ein Verbindungsstück, ein Fersenkissen (18), ein erstes und ein zweites Federelement (40, 42) und einen Abstandshalter (46);
Anordnen des ersten und zweiten Federelements (40, 42), wobei Teile des zweiten Federelements (42) unter dem ersten Federelement (40) positioniert sind;
Verbinden der distalen Enden des ersten und zweiten Federelements (40, 42) miteinander;
Anordnen des Abstandshalters (46) zwischen den proximalen Enden der ersten und zweiten Federelemente (40, 42), wobei die ersten und zweiten Federelemente (40, 42) voneinander beabstandet sind, wenn sich der Prothesenfuß in einer Ruheposition befindet;
Verbinden der proximalen Enden des ersten und zweiten Federelements (40, 42) mit dem Verbindungsstück (16) mit mindestens einem Befestigungselement;
das distale Ende des zweiten Federelements (42) wird mit einem Zehenendabschnitt der Basisfeder (12) verbunden, wobei die distalen Enden des ersten und zweiten Federelements (40, 42) im Wesentlichen parallel zu einer oberen Fläche der Basisfeder (12) angeordnet sind;
Anbringen des Fersenkissens (18) an der Basisfeder (12) an einer Stelle, die von einem Fersenende der Basisfeder (12) nach vorne beabstandet ist, wobei das Fersenkissen (18) so angeordnet ist, dass es eine Bodenfläche des zweiten Federelements (42) berührt, **dadurch gekennzeichnet, dass** das Verfahren ferner umfasst:
Anordnen des ersten und zweiten Federelements (40, 42) parallel zueinander.

6. Verfahren nach Anspruch 5, **dadurch gekennzeichnet, dass** es ferner umfasst:
Bereitstellen des ersten und zweiten Federelements (40, 42) mit einem unteren Abschnitt (54, 66) und einem oberen Abschnitt (56, 68);
Anordnen des unteren Teils (54, 66) in einem Winkel von etwa 5° bis etwa 30° relativ zu einer horizontalen Ebene;
Anordnen des oberen Teils (56, 68) im Wesentlichen vertikal.

7. Verfahren nach Anspruch 5, **dadurch gekennzeichnet, dass** die Basisfeder (12) einen in dem Zehenendabschnitt ausgebildeten Sandalenschlitz aufweist und dass das Verbinden des distalen Endes des zweiten Federelements (42) mit der Basisfeder (12) das Verkleben mit der Basisfeder (12) an einer Stelle hinter dem Sandalenschlitz umfasst.

8. Verfahren nach Anspruch 5, **dadurch gekennzeichnet, dass** ein Teil des ersten Federelements (40) während der Benutzung des Prothesenfußes in Kontakt mit dem zweiten Federelement (42) gebracht werden kann.

## Revendications

1. Pied prothétique, comprenant :
un ressort de base (12) ayant une partie d'extrémité côté orteils et une partie d'extrémité côté talon ;
un ensemble de ressort supérieur (14), comprenant :
un premier élément de ressort (40) ayant une extrémité distale et une extrémité proximale ;
un deuxième élément de ressort (42) ayant une extrémité distale et une extrémité proximale ;
une première connexion prévue entre les extrémités distales des premier et deuxième éléments de ressort (40, 42), et une deuxième connexion prévue entre l'extrémité distale du deuxième élément de ressort (42) et une surface supérieure du ressort de base (12) ;
une fente s'étendant vers l'arrière depuis les extrémités distales des premier et deuxième éléments de ressort (40, 42) sur au moins la moitié de la longueur de la première partie (54, 66) de l'ensemble de ressort supérieur (14) ;
un connecteur (16) relié aux extrémités proximales des premier et deuxième éléments de ressort (40, 42) et configuré pour relier le pied prothétique à une prothèse de membre inférieur ;
un coussin de talon (18) monté sur le ressort de base (12) à un endroit situé à l'avant de l'extrémité côté talon du ressort de base (12), le coussin de talon (18) étant agencé pour entrer en contact avec une surface inférieure du deuxième élément de ressort (40) pendant l'utilisation du pied prothétique,
**caractérisé en ce que**
le deuxième élément de ressort (42) s'étend de manière sensiblement parallèle et à distance du premier élément de ressort (40) sensiblement sur toute la longueur du premier élément de ressort (40) ;
une première partie (54, 66) des premier et deuxième éléments de ressort (40, 42) est agencée selon un premier angle par rapport à un plan horizontal ; et
une deuxième partie (56, 68) des premier et deuxième éléments de ressort (40, 42) s'étend à partir de la première partie (54, 66).

2. Pied prothétique selon la revendication 1,
**caractérisé en ce que** la première connexion est assurée par un adhésif.

3. Pied prothétique selon la revendication 1,
**caractérisé en ce que** le deuxième élément de ressort (42) est espacé du premier élément de ressort (40) par des éléments d'espacement (44, 46) placés aux extrémités distales et proximales des premier et deuxième éléments de ressort (40, 42).

4. Pied prothétique selon la revendication 1,
**caractérisé en ce que** l'épaisseur du premier élément de ressort (40) s'élève jusque 15 % de l'épaisseur du deuxième élément de ressort (42).

5. Procédé de fabrication d'un pied prothétique selon la revendication 1, consistant à :
fournir un ressort de base (12), un connecteur, un coussin de talon (18), des premier et deuxième éléments de ressort (40, 42) et un élément d'espacement (46) ;
placer les premier et deuxième éléments de ressort (40, 42), des parties du deuxième élément de ressort (42) étant positionnées sous le premier élément de ressort (40) ;
relier les extrémités distales des premier et deuxième éléments de ressort (40, 42) l'une à l'autre ;
placer élément d'espacement (46) entre les extrémités proximales des premier et deuxième éléments de ressort (40, 42), les premier et deuxième éléments de ressort (40, 42) étant espacés lorsque le pied prothétique est dans une position de repos ;
relier les extrémités proximales des premier et deuxième éléments de ressort (40, 42) au connecteur (16) à l'aide d'au moins un élément de fixation ;
relier l'extrémité distale du deuxième élément de ressort (42) à une partie d'extrémité côté orteils du ressort de base (12), les extrémités distales des premier et deuxième éléments de ressort (40, 42) étant disposées sensiblement parallèlement à une surface supérieure du ressort de base (12);
monter le coussin de talon (18) sur le ressort de base (12) à un endroit situé à l'avant de l'extrémité côté talon du ressort de base (12), le coussin de talon (18) étant disposé de manière à entrer en contact avec une surface inférieure du deuxième élément de ressort (42),
**caractérisé en ce que** le procédé consiste en outre à :
placer les premier et deuxième éléments de ressort (40, 42) parallèlement l'un à l'autre.

6. Procédé selon la revendication 5,
**caractérisé en ce qu'**il consiste en outre à :
équiper les premier et deuxième éléments de ressort (40, 42) d'une partie inférieure (54, 66) et d'une partie supérieure (56, 68) ;
placer la partie inférieure (54, 66) selon un angle d'environ 5° à environ 30° par rapport à un plan horizontal ;
placer la partie supérieure (56, 68) sensiblement verticalement.

7. Procédé selon la revendication 5,
**caractérisé en ce que** le ressort de base (12) comprend une fente de sandale formée dans la partie d'extrémité côté orteils, et la connexion de l'extrémité distale du deuxième élément de ressort (42) au ressort de base (12) inclut le collage au ressort de base (12) à un endroit espacé postérieurement à la fente de sandale.

8. Procédé selon la revendication 5,
**caractérisé en ce qu'**une partie du premier élément de ressort (40) est mobile en contact avec le deuxième élément de ressort (42) pendant l'utilisation du pied prothétique.
